# EUROPEAN PATENT APPLICATION

(11) **EP 1 484 609 A1**
(43) Date of publication of application: **08.12.2004**
(21) Application number: 03712682.8
(22) Date of filing: 13.03.2003
(51) Int. Cl.: G01N 30/88, B01D 15/08, C12Q 1/68

(54) **METHOD OF PURIFYING OXIDATIVELY INJURED GUANINE NUCLEOSIDE, METHOD OF MEASURING THE SAME AND ANALYZER FOR THE EMBODIMENT THEREOF**

(30) Priority: 14.03.2002 JP 2002070836
(71) Applicant: Kasai, Hiroshi, Kitakyushu-shi, Fukuoka 807-0805 (JP)
(72) Inventor: Kasai, Hiroshi, Kitakyushu-shi, Fukuoka 807-0805 (JP)
(74) Representative: Dealtry, Brian
(86) International application number: PCT/JP2003/003007
(87) International publication number: WO 2003/076925

(57) **Abstract**

An object of the invention is to provide a purification method for oxidatively damaged guanine nucleosides having high accuracy and reproducibility, and also for which consideration is given to economic efficiency and environmental aspects, a measuring method therefor, and an analyzer for performing such.

The purification method for oxidatively damaged guanine nucleosides is a purification method for oxidatively damaged guanine nucleosides generated as a result of guanine damage in DNA or RNA, comprising a first purification step for purifying oxidatively damaged guanine nucleosides contained in a sample by anion-exchange chromatography. The purification method for 8-OH-dG is a purification method for 8-OH-dG contained in a sample, wherein 8-OH-rGuo is previously added to the sample so as to purify it. The measuring method for oxidatively damaged guanine nucleosides comprises a measuring step for measuring the purified oxidatively damaged guanine nucleosides obtained by the purification method.

## Description

### TECHNICAL FIELD

The present invention relates to a purification method for oxidatively damaged guanine nucleosides, particularly a purification method for 8-hydroxydeoxyguanosines (hereunder, abbreviated 8-OH-dG), a measuring method therefor, and an analyzer for performing such.

### BACKGROUND ART

Recently, the effect of active oxygen in vivo has been well researched. Normally, active oxygen acts as a defense system when a foreign body is invading into a living organism. However, if excessive active oxygen is generated due to food additives (carcinogen), air pollution, smoking, stress, and the like, it causes DNA damage and produces 8-OH-dG which is a kind of oxidative DNA damaged product. This 8-OH-dG is considered to induce mutation and play an important role in the carcinogenesis process. Moreover, attention has been paid to the active oxygen as a causative factor not only of carcinogenesis but also of various disorders or aging.

Therefore, by knowing the amount of active oxygen in vivo, individual carcinogenesis risk evaluation, prediction and diagnosis of various disorders, evaluation of the degree of aging or general health can be performed.

However, since the active oxygen in vivo is unstable, it is difficult to directly detect this. Therefore, as an index of active oxygen, it has been proposed to measure the 8-OH-dG produced by the active oxygen.

Analysis methods of 8-OH-dG reported so far can be largely classified into six types, including; (1) a method of analyzing by HPLC-ECD, a fraction which was purified by an affinity column having antibodies against 8-OH-dG, (2) a method of connecting three or four columns and using a column switching method to finally detect 8-OH-dG by HPLC-ECD, (3) a method of directly analyzing urine by the ELISA method, (4) a method by a GC-MS (requiring an internal standard substance), (5) a method by an LC-MS-MS (requiring an internal standard substance), and (6) a method of connecting a multifunction column (a gel filter column having both functions of reverse phase column and cation-exchange column) and a reverse phase column through a sampling injector (an apparatus which collects a specific fraction and injects into columns after mixing), so as to detect by ECD.

However, in method (1), due to the low recovery rate, it is required to use a radioactive internal standard substance.

In method (2), the pretreatment is complicated. Moreover, it is difficult to determine the timing for switching valves and impurities are often found in the vicinity of the peak of 8-OH-dG in many cases. Furthermore, since not only a large amount of eluent and washing solution is required but also a large amount of poisonous effluent is generated, it is not preferable from an environmental aspect.

In method (3), since there is a problem in the specificity, the reproducibility is inferior.

In methods (4) and (5), since the measuring equipment is expensive, it is not preferable in terms of economic efficiency. Furthermore, since the recovery rate is unstable, internal standard substances are required, however they are difficult to obtain.

In method (6), since the analyzing time per one specimen is as long as 165min, the mass treatment by continuous operation is difficult. Furthermore, when analyzing by chromatography, impurities overlapping the peak of 8-OH-dG are very likely to be detected. Therefore, the measurement accuracy is insufficient in any of the methods. An object of the present invention is to provide a purification method for oxidatively damaged guanine nucleosides, particularly 8-OH-dG, having high accuracy and reproducibility, and also for which consideration is given to economic efficiency and environmental aspects, a measuring method therefor, and an analyzer for performing such.

### DISCLOSURE OF INVENTION

The present inventor found that oxidatively damaged guanine nucleosides such as 8-OH-dG, 8-hydroxyguanosine (ribonucleoside) (hereunder, abbreviated 8-OH-rGuo), or the like can be specifically absorbed and recovered by using an anion-exchange column. Furthermore, regarding 8-OH-dG, he found that 8-OH-dG can be accurately fractionated by using 8-OH-rGuo as an internal standard marker, consequently 8-OH-dG can be measured with high accuracy and reproducibility, and in addition, it is superior in terms of economic efficiency and environmental aspects. From these findings he has completed the present invention.

That is, the purification method for oxidatively damaged guanine nucleosides of the present invention is a purification method for oxidatively damaged guanine nucleosides generated as a result of guanine damage in DNA or RNA, comprising a first purification step for purifying oxidatively damaged guanine nucleosides contained in a sample by anion-exchange chromatography. Moreover, the oxidatively damaged guanine nucleoside is preferably 8-OH-dG. The purification method for 8-OH-dG of the present invention is a purification method for 8-hydroxydeoxyguanosines (8-OH-dG) contained in a sample, wherein 8-hydroxyguanosines (ribonucleosides) (8-OH-rGuo) are previously added to the sample as an internal standard marker for 8-OH-dG so as to purify it. The purification method for 8-OH-dG of the present invention is a purification method for 8-hydroxydeoxyguanosines (8-OH-dG) contained in a sample, wherein 8-hydroxyguanosine (ribonucleosides) (8-OH-rGuo) is previously added to the sample, comprising a first purification step for purifying the sample by anion-exchange chromatography, and a second purification step for further purifying the fraction containing 8-OH-dG obtained in the first purification step by reverse phase chromatography. In the purification method for oxidatively damaged guanine nucleosides, the sample is preferably urine. Moreover, in the purification method for 8-OH-dG, the sample is preferably urine. The measuring method for oxidatively damaged guanine nucleosides of the present invention comprises a measuring step for measuring purified oxidatively damaged guanine nucleosides obtained by the purification method. The measuring method for 8-OH-dG of the present invention comprises a measuring step for measuring purified 8-OH-dG obtained by the purification method. In the measuring method for 8-OH-dG of the present invention, the purified 8-hydroxydeoxyguanosines (8-OH-dG) are measured in anion-exchange chromatography in the order of; (1) peak recognition of ribonucleosides 8-OH-rGuo, (2) starting of 8-OH-dG fractionation after a fixed time, (3) finishing of 8-OH-dG fractionation after a fixed time, and (4) optionally mixing 8-OH-dG fraction, and then injected into a reverse phase column.

The analyzer of the present invention is an apparatus for purifying and measuring 8-hydroxydeoxyguanosines (8-OH-dG), comprising; an anion-exchange column (HPLC-1) which specifically absorbs 8-OH-dG contained in a sample, a UV detector which detects an elution position of 8-hydroxyguanosine (ribonucleoside) (8-OH-rGuo), a reverse phase column (HPLC-2) which further purifies the fraction containing 8-OH-dG obtained from the anion-exchange column (HPLC-1), and a detector which measures the purified 8-OH-dG obtained from the reverse phase column (HPLC-2).

The control program of the present invention is a program for controlling a process for recovering 8-hydroxydeoxyguanosines (8-OH-dG) contained in a sample by column chromatography, which executes on a computer processes for: receiving a peak signal of a marker (8-OH-rGuo) previously added to the sample from a UV detector; outputting a signal to open a valve connected to a sampler, during 8-OH-dG elution after a fixed time; starting fractionation; and outputting a fractionation termination signal after another fixed time; and then outputting a signal to inject the obtained 8-OH-dG fraction into a second purifying column; thereby purifying and recovering a detected substance (8-OH-dG) eluted from the column.

### BRIEF DESCRIPTION OF THE DRAWINGS

FIG. 1 is a schematic diagram showing an embodiment of an apparatus for purifying and measuring 8-OH-dG.
FIG. 2 is a schematic diagram showing an embodiment of an apparatus for purifying and measuring 8-OH-dG.
FIG. 3 shows an example of a separation pattern of a mixture of urine, 8-OH-dG and 8-OH-rGuo, using an anion-exchange column (HPLC-1), showing a positional validation of the markers.
FIG. 4 shows an example of a separation pattern of human urine using an anion-exchange column (HPLC-1).
FIG. 5 shows an example of a separation pattern of human urine using a reverse phase column (HPLC-2).
FIG. 6 shows an example of a separation pattern of human urine using an anion-exchange column (HPLC-1).
FIG. 7 shows an example of a separation pattern of human urine using a reverse phase column (HPLC-2).
FIG. 8 shows an example of a separation pattern of rat urine using a reverse phase column (HPLC-2).
FIG. 9 shows an example of a separation pattern of rat urine using a reverse phase column (HPLC-2).

### BRIEF DESCRIPTION OF THE REFERENCE SYMBOLS

- 11.: Anion-exchange column (HPLC-1)
- 12.: Reverse phase column (HPLC-2)
- 13.: Detector
- 14.: UV detector
- 15.: Switching valve
- 16.: Switching valve
- 17.: Automatic sampler
- 27.: Sampling injector

### BEST MODE FOR CARRYING OUT THE INVENTION

The present invention is a purification method for accurately measuring oxidatively damaged guanine nucleosides being the index of active oxygen, particularly 8-OH-dG, a measuring method therefor, and an analyzer for performing such, in order to evaluate the amount of active oxygen in vivo.

The oxidatively damaged nucleoside including 8-OH-dG is generated as a result of DNA or RNA damage by active oxygen (oxygen radical) and the like in vivo, and is used as the index of active oxygen. The oxidatively damaged nucleosides besides 8-OH-dG include 2-hydroxydeoxyadenosine (2-OH-dA), 5-hydroxydeoxycytidine (5-OH-dC), 5-formyldeoxyuridine (5-CHO-dU), 8-OH-rGuo, and the like. These oxidatively damaged nucleosides are excreted out of the organism as undesired substance in urine. Moreover, among them, oxidatively damaged guanine nucleosides such as 8-OH-dG, 8-OH-rGuo or the like are negatively charged so that they are easily purified and recovered by an anion-exchange column described in the following paragraph. Among them, it is preferable to use specifically 8-OH-dG for the index of active oxygen. The oxidatively damaged guanine nucleoside in the present application is generated as a result of guanine damage in DNA or RNA by active oxygen (oxygen radical), and oxidatively damaged means hydroxylation.

Samples used for the purification method and the measuring method for oxidatively damaged guanine nucleosides (including 8-OH-dG) of the present invention includes all biological samples such as urine, serum, cerebrospinal fluid, saliva, the medium after culturing cells, and the like. Among them, urine is particularly preferable since it is easy to collect and the oxidatively damaged guanine nucleosides are stable therein.

Hereunder is a description of a purification method, a measuring method, and an analyzer for performing such, according to the present invention, in relation to 8-OH-dG.

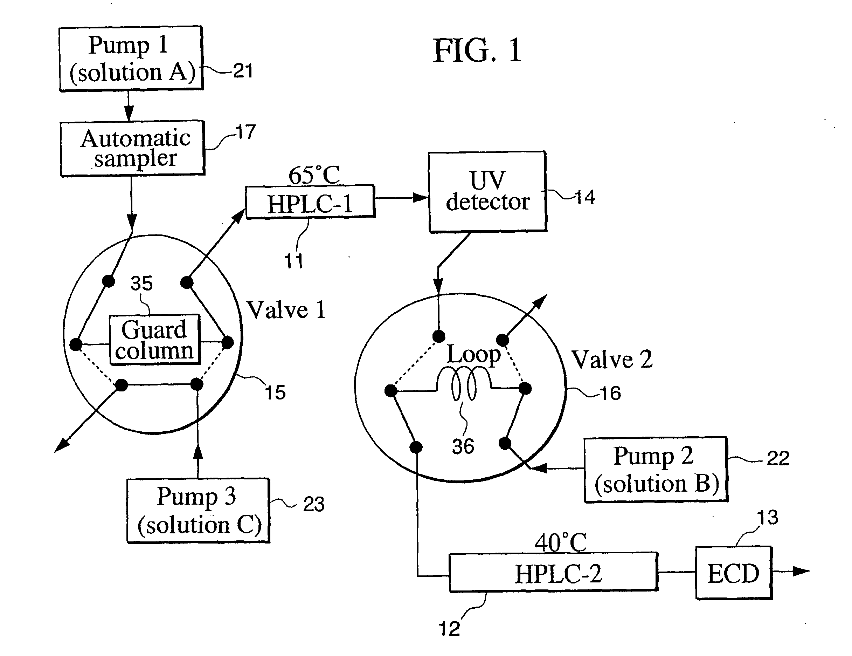

An apparatus for purifying and measuring 8-OH-dG according to an embodiment of the present invention comprises; an anion-exchange column (HPLC-1) which specifically absorbs 8-OH-dG, a UV detector which detects 8-OH-rGuo being an index of the elution position of 8-OH-dG, a reverse phase column (HPLC-2) which further purifies the fraction containing 8-OH-dG obtained by the anion-exchange column (HPLC-1), and a detector which measures the purified 8-OH-dG obtained by the reverse phase column (HPLC-2). FIG. 1 is a schematic diagram showing an example of an analyzer. In the diagram, reference symbol 11 is an anion-exchange column (HPLC-1). This is connected to a reverse phase column (HPLC-2) 12 via a UV detector 14 and a column switching valve 16. Moreover, upstream of the anion-exchange column (HPLC-1) 11 is connected a column switching valve 15 to which is connected an automatic sampler 17 for injecting samples.

Furthermore, pumps 21, 22 and 23 are provided for sending solution A, B and C to the respective columns. The solution A and B are eluents for eluting molecules absorbed in the columns (an eluent used for the anion-exchange column (HPLC-1) 11 is solution A and an eluent used for the reverse phase column (HPLC-2) 12 is solution B). The solution C is a washing solution for washing a guard column (filled with an anion-exchange resin which is the same as used in the anion-exchange column (HPLC-1) 11) connected to the column switching valve 15. The pump 21 is connected to the automatic sampler 17. The pump 22 is connected to the column switching valve 16. The pump 23 is connected to the column switching valve 15.

In FIG. 1, instead of the automatic sampler 17, a sampling injector ("231XL" manufactured by Gilson) having a function to automatically operate the column switching valve 16 by peak detection of 8-OH-rGuo, may be used.

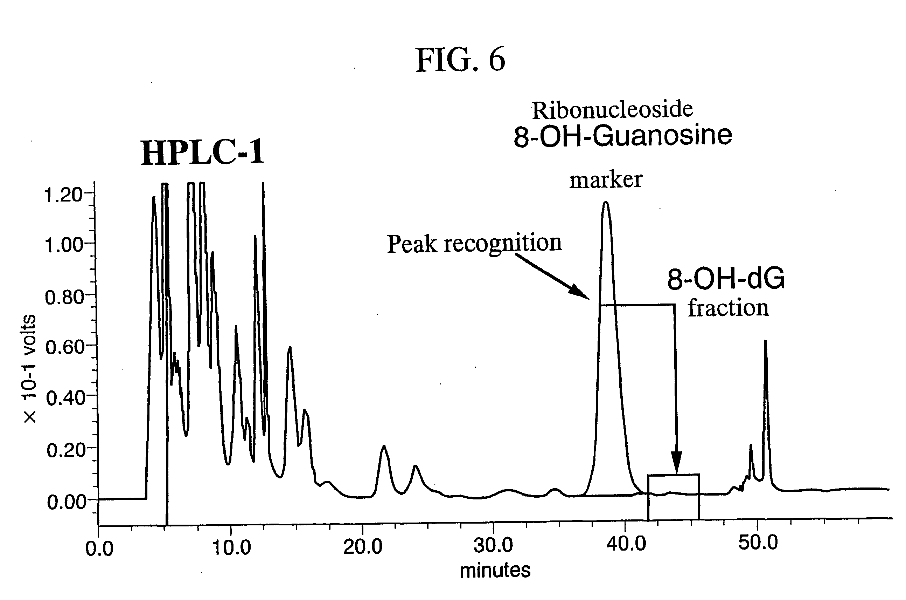

In order to perform this method, a new program was loaded into the 231XL and the measurement performed. This program performs (1) peak recognition of ribonucleosides 8-OH-rGuo, (2) starting of 8-OH-dG fractionation at a fixed time, (3) finishing of 8-OH-dG fractionation at a fixed time, and (4) injection into the HPLC-2 (refer to FIG. 6). In more detail, these functions are realized by the following flows.
(1) A sample is injected into the HPLC-1 by the 231XL.
(2) The system is kept standing-by for a preset time (T1).
(3) The 231XL starts monitoring the signal from the UV detector.
(4) The system is kept standing-by until exceeding the preset UV level (peak detection).
(5) After the peak detection, the system is kept standing-by for a preset time (T2). Then a contact signal is output to a valve so as to start fractionation inside the loop.
(6) After a preset time (T3), the contact signal is output to the valve. Then, fractionation is finished and at the same time the fraction inside the loop is injected into HPLC-2.

As described later, according to the sampling injector ("231XL" manufactured by Gilson), the fractionation range (time) of 8-OH-dG is automatically determined based on the relative position with respect to 8-OH-rGuo. Therefore it is not necessary to preset the fractionation range (time) of 8-OH-dG.

Since the abovementioned anion-exchange column (HPLC-1) 11 specifically absorbs 8-OH-dG contained in the sample, the recovery rate is very high and almost all impurities can be removed, so that fractions with less impurities can be obtained. Moreover, as described above, according to the anion-exchange column (HPLC-1) 11, negatively charged oxidatively damaged guanine nucleosides such as 8-OH-rGuo or the like can be easily purified and recovered. The anion-exchange column (HPLC-1) 11 is not specifically limited provided an anion-exchange resin is used for the filler. Examples of specific filler include styrenedivinylbenzene polymer with quaternary ammonium group, polyhydroxymethacrylate polymer with quaternary ammonium group, and the like. Moreover, examples of commercial filler include Aminex HPX-72S (manufactured by Bio-Rad), Shodex column filler (manufactured by Showa Denko K.K.), MCI GEL CA08F (manufactured by Mitsubishi Chemical Industries Ltd., Hamilton RCX-10), and the like.

Moreover, the particle diameter of the anion-exchange resin is preferably from 7 to 12µm.

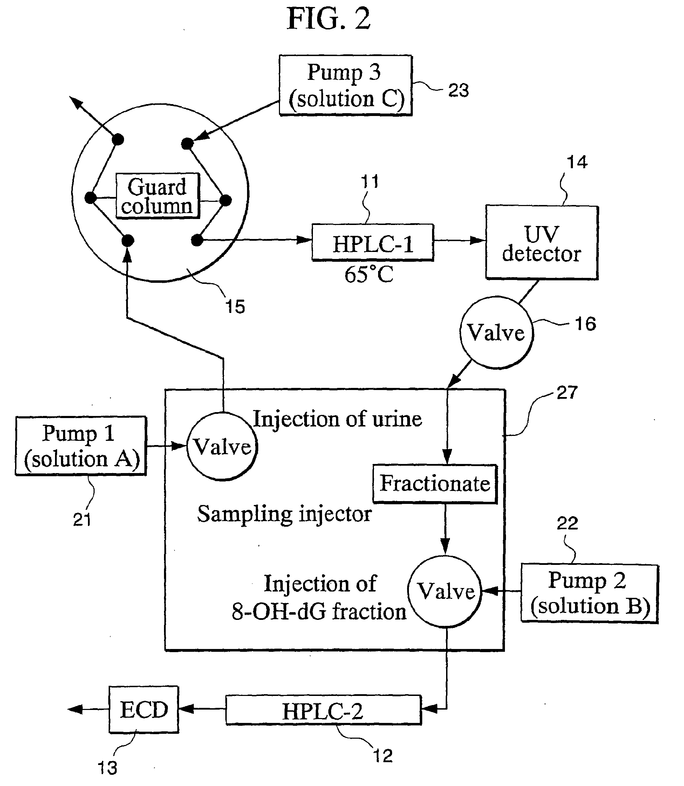

The internal diameter of the column which is filled with the anion-exchange resin is not specifically limited, however preferably this is from about 1mm to 1.5mm. In the case where the internal diameter of the column is from 2.0 to 4.6mm as shown in FIG. 2, it is preferable to use a sampling injector 27 ("233XL" manufactured by Gilson, or the like) connected to the column switching valve 16 so that the fraction containing 8-OH-dG is automatically injected into the reverse phase column (HPLC-2) 12. In order to perform this method, a new program was loaded into the 233XL and the measurement performed. This program performs (1) peak recognition of ribonucleosides 8-OH-rGuo, (2) starting of 8-OH-dG fractionation at a fixed time, (3) finishing of 8-OH-dG fractionation at a fixed time, (4) mixing of 8-OH-dG fraction, and (5) injection into the HPLC-2. In more detail, these functions are realized by the following flows.
(1) A sample is injected into the HPLC-1 by the 233XL.
(2) The system is kept standing-by for a preset time (T1).
(3) The 233XL starts monitoring the signal from the UV detector.
(4) The system is kept standing-by until exceeding the preset UV level (peak detection).
(5) After the peak detection, the system is kept standing-by for a preset time (T2). Then fractionation inside the 233XL vial tubes is started.
(6) After a preset time (T3), the fractionation is finished.
(7) The obtained fraction is stirred by drawing and discharging.
(8) The fraction is partly injected into the HPLC-2.

The length of the column which is filled with the anion-exchange resin is not specifically limited. However the column may be shortened according to the particle diameter of the anion-exchange resin, the exchange capacity, or the like, so as to shorten the analysis time.

The abovementioned UV detector 14 monitors the fraction eluted out from the anion-exchange column (HPLC-1) 11, and detects the elution position of 8-OH-dG contained in the sample. In this manner, by monitoring the elution position of 8-OH-rGuo by the UV detector 14, the elution time of 8-OH-dG can be obtained. Together with the above, by operating the column switching valve 16, the fraction containing 8-OH-dG can be reliably collected.

The abovementioned reverse phase column (HPLC-2) 12 further purifies the fraction containing 8-OH-dG obtained from the anion-exchange column (HPLC-1). This column is not specifically limited as long as it has the property of a reverse phase column. Examples of commercial products include YMC-Pack ODS-AM (S-5µm) (manufactured by YMC Co., Ltd.), Shiseido Capcell Pac C18 MG (S-5µm) (manufactured by Shiseido Co. Ltd.), and the like.

The abovementioned detector 13 measures the purified 8-OH-dG obtained by the reverse phase column (HPLC-2), and is provided downstream of the reverse phase column (HPLC-2) 12. For the detector 13, an Electrochemical detector (ECD), a liquid chromatography mass spectrometry (LCMS), and the like may be used. Moreover, concerning the Electrochemical detector (ECD), the peak of 8-OH-dG appears in a characteristic ratio by selecting two kinds of preset voltages, so that the peak can be identified to be 8-OH-dG.

Moreover, the analyzer for purifying and measuring 8-OH-dG according to the embodiment of the present invention can treat a large amount of samples by continuous operation. In this case, the washing solution (solution C) of the guard column 35 is preferably a composition of 0.5M ammonium sulfate : acetonitrile = about 7:3.

As described above, according to the 8-OH-dG analyzer according to the embodiment of the present invention, the anion-exchange column (HPLC-1) 11 specifically absorbs 8-OH-dG contained in the sample, and removes almost all impurities contained in the sample at once. Moreover, based on the elution position of 8-OH-rGuo detected by the UV detector 14, the purified 8-OH-dG can be reliably fractionated so that it can be superior in the recovery rate and the reproducibility. Furthermore, by continuous operation a large amount of samples can be treated. Since the analyzer is relatively low in price, it is also superior in terms of economic efficiency.

Hereunder is a description of the purification method and the measuring method for 8-OH-dG using the analyzer shown in FIG. 1.

### (Determination of fractionation range (time))

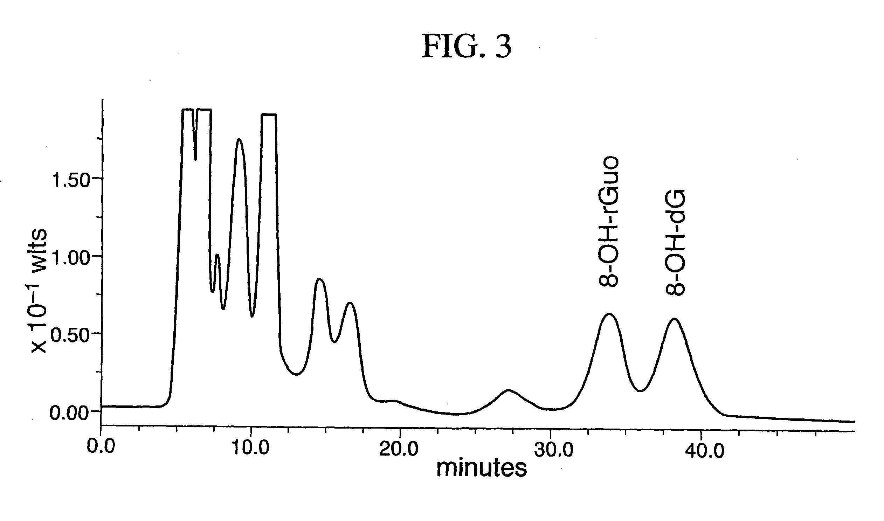

A mixture of 8-OH-dG, 8-OH-rGuo, and urine was injected into the analyzer shown in FIG. 1 so as to previously determine the fractionation range of 8-OH-dG. FIG. 3 shows an example of a separation pattern of the mixture of 8-OH-dG, 8-OH-rGuo, and urine, showing a positional validation of 8-OH-rGuo being an internal standard marker of 8-OH-dG. In this manner, by previously determining the fractionation range, an accurate elution time for 8-OH-dG can be obtained. Together with the above, by setting so as to operate the column switching valve 16, the fraction containing 8-OH-dG can be reliably collected. As described above, in FIG. 1, if the sampling injector (231XL) is used instead of the automatic sampler 17, since the fractionation range (time) of 8-OH-dG is automatically determined by the peak detection based on the relative position with respect to 8-OH-rGuo, it is not necessary to preset the fractionation range (time) of 8-OH-dG.

### (Purification Method)

The purification method for 8-OH-dG of the present invention comprises a first purification step for purifying the sample by anion-exchange chromatography. Moreover, as described above, negatively charged oxidatively damaged guanine nucleosides such as 8-OH-rGuo or the like as well as 8-OH-dG can be easily purified and recovered by the anion-exchange chromatograph.

The elution conditions in the first purification step are preferably such that, when the column temperature is from 60 to 65°C and the internal diameter of the column is 1mm, the flow rate is from 17 to 20µl/min.

In the purification method for 8-OH-dG of the present invention, it is preferable to previously add 8-OH-rGuo to the sample as the internal standard marker for 8-OH-dG, so as to purify it. If the 8-OH-rGuo is previously added to the sample, the 8-OH-dG is eluted at a fixed time after the elution of the 8-OH-rGuo. Accordingly, by monitoring the elution position of 8-OH-rGuo by the UV detector 14, the accurate elution position (time) of 8-OH-dG can be obtained, so that the fraction containing 8-OH-dG can be reliably collected.

Moreover, in the purification method for 8-OH-dG of the present invention, it is preferable to previously add 8-OH-rGuo to the sample as the internal standard marker for 8-OH-dG so as to perform the first purification step by anion-exchange chromatography, and to further purify the fraction containing 8-OH-dG obtained in the first purification step (second purification step).

For the second purification step, it is preferable to purify by reverse phase chromatography. Since the eluent (solution B) used for the reverse phase chromatography, the temperature condition, and the like vary depending on the reverse phase column (HPLC-2) 12 to be used, these are appropriately determined. For example, in the case where human urine is analyzed using the YMC-Pack ODS-AM (S-5µm) (manufactured by YMC Co., Ltd.) as the reverse phase column, preferably, the column temperature is about 40°C, and the flow rate is about 0.9ml/min.

### (Measuring Method)

The measuring method of the present invention comprises a measuring step for measuring the amount of the purified 8-OH-dG obtained by the purification method described above, wherein the abovementioned Electrochemical detector (ECD), a liquid chromatography mass spectrometry (LCMS), and the like may be used for measuring the amount of the purified 8-OH-dG. The measuring method is applicable for measuring the purified oxidatively damaged guanine nucleosides such as 8-OH-rGuo or the like as well as 8-OH-dG.

Moreover, in the case of continuous operation, both in the apparatus comprising the automatic sampler 17 as shown in FIG. 1 and in the apparatus comprising the sampling injector 27 as shown in FIG. 2, the elution position of 8-OH-dG is preferably checked regularly.

As described above, according to the purification method for the oxidatively damaged guanine nucleosides of the present invention, the purified oxidatively damaged guanine nucleosides can be obtained with high recovery rate. Furthermore, since the flow rate of the anion-exchange column (HPLC-1) in the first purification step is very low, the consumption of the eluent (solution A and solution B) and the washing solution (solution C) is extremely small, and the amount of the effluent after purification is small, so that the method is also preferable from the aspect of environmental protection. According to the purification method for 8-OH-dG of the present invention, the purified 8-OH-dG can be reliably fractionated and a fraction with less impurities near the peak of 8-OH-dG can be obtained. Moreover, even in the case of continuous operation, by the peak detection of 8-OH-rGuo, it becomes possible to correspond to the displacement of the fraction range of each sample, so that the fraction containing 8-OH-dG can be reliably collected. Moreover, since the measuring method of the present invention measures the purified oxidatively damaged guanine nucleosides such as purified 8-OH-dG, 8-OH-rGuo or the like obtained by the above purification method, it has high accuracy and reproducibility.

The scope of the techniques of the present invention is not limited to the above embodiments. Various modifications can be made without departing from the sprit or scope of the present invention. For example, the composition of the eluent (solution A and solution B) and the washing solution (solution C), or the like may be appropriately modified corresponding to the columns (fillers) to be used.

The measuring method for oxidatively damaged guanine nucleosides of the present invention may be used in individual carcinogenesis risk evaluation, prediction and diagnosis of various disorders related to active oxygen (for example diabetes), evaluation of degree of aging or general health.

The evaluation method for the results obtained by the measuring method, is described below using an example in the case of 8-OH-dG. As well as the urine sample, 8-OH-dG standard solution was injected into the analyzer periodically. Then, these peak areas were compared to calculate the 8-OH-dG concentration in the sample. The calculated 8-OH-dG concentration was then divided by the concentration of a standard substance such as creatinine, or calculated as the amount of 8-OH-dG in the urine for 24 hours.

### Examples

Hereunder is a specific description of the present invention using examples, however the present invention is not to be considered as limited to these.

### (Example 1)

### <Preparation of urine sample 1>

1ml of human urine was placed in each of two Eppendorf tubes and frozen at -20°C. The frozen urine was thawed and homogenized. 100µl of each homogenate was diluted with the same volume of slightly acidic solution (composition; 96ml of 0.6mM sulfuric acid and 4ml of acetonitrile). Consequently, 12µg of 8-OH-dG and 12µg of 8-OH-rGuo were added. The pH was adjusted below 7 by adding 6.7µl of 2M sodium acetate (pH4.5). The mixture was well stirred and centrifuged at 15,000 rpm for 5 minutes. The supernatant was made the urine sample 1.

10µl of the urine sample 1 prepared as described above was purified by an anion-exchange column (particle diameter of filler (manufactured by Bio-Rad) 12µm, internal diameter 1mm, guard column length 4cm, and main column length 12cm). The separation pattern is shown in FIG. 3. The separation pattern was obtained using a UV detector ("UV-8020" manufactured by Tosoh Corp.) (absorption wavelength 254nm).

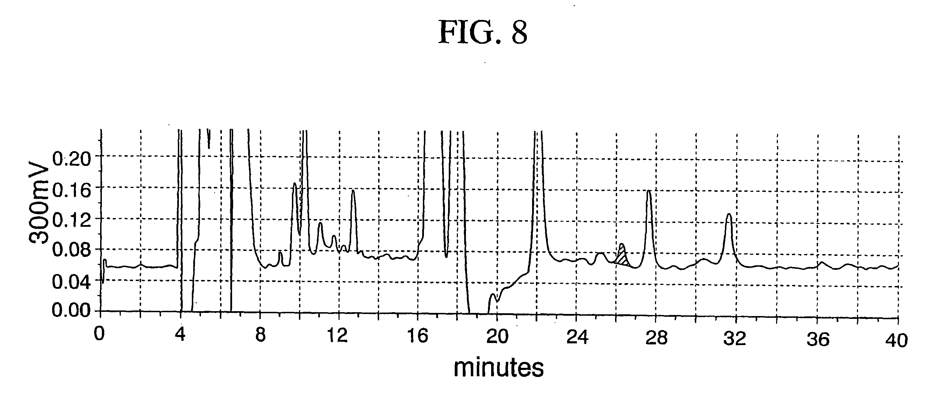

As shown in FIG. 3, 8-OH-dG was eluted at a fixed time after the 8-OH-rGuo elution. Therefore, by monitoring the 8-OH-rGuo elution the accurate elution position of 8-OH-dG could be ascertained so that the fraction containing 8-OH-dG could be reliably obtained.

### (Example 2)

### <Preparation of urine sample 2>

1ml of human urine was placed in each of two Eppendorf tubes and frozen at -20°C. The frozen urine was then thawed and homogenized. 100µl of each homogenate was diluted with the same volume of slightly acidic solution (composition; 96ml of 0.6mM sulfuric acid and 4ml of acetonitrile). Consequently, 12µg of 8-OH-rGuo was added. The pH was adjusted below 7 by adding 6.7µl of 2M sodium acetate (pH4.5). The mixture was well stirred and centrifuged at 15,000 rpm for 5 minutes. The supernatant was made the urine sample 2.

### <Purification by anion-exchange chromatography>

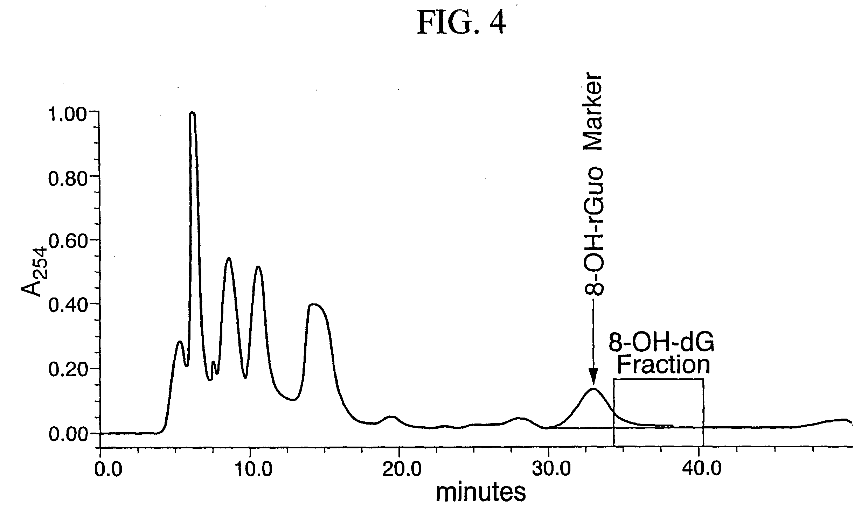

The human urine sample 2 was purified using an anion-exchange column to obtain a fraction containing 8-OH-dG (34 to 41 min). 0.3mM sulfuric acid, 2% acetonitrile eluent was used as a solution A. The column temperature was 65°C and the flow rate was 17µl/min. The separation pattern is shown in FIG. 4. The anion-exchange column was made using a filler of an Aminex HPX-72S column (manufactured by Bio-Rad) (300x7.8mm, particle diameter 12µm, degree of cross-linkage 8%, and sulfate type) refilled in a column of an internal diameter of 1mm (guard column length 4cm, and main column length 12cm). The separation pattern was obtained using the UV detector ("UV-8020" manufactured by Tosoh Corp.) (absorption wavelength 254nm).

### <Purification by reverse phase chromatography>

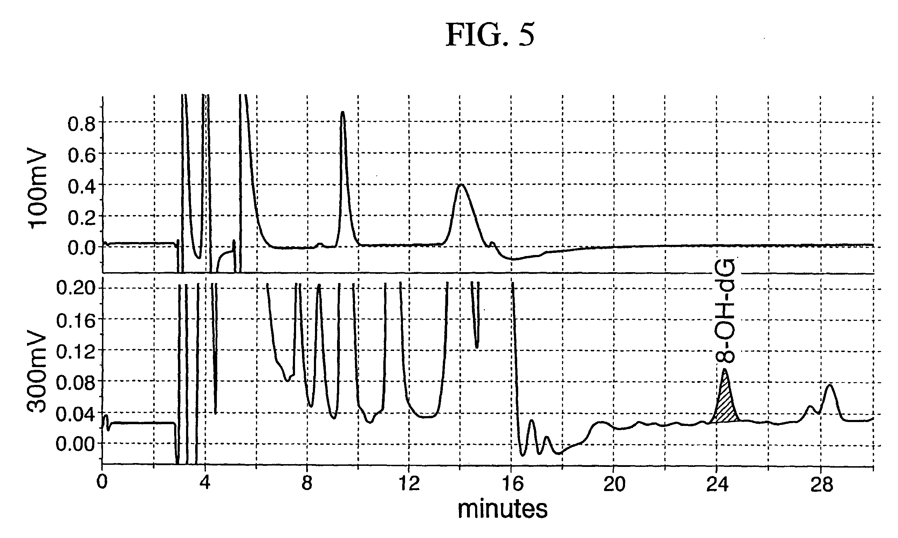

119µl of the fraction containing 8-OH-dG (34 to 41 min) obtained by the anion-exchange chromatography was automatically injected into a reverse phase chromatography. A Shiseido Capcell Pak C18 MG (S-5µm) (250x4.6mm) was used for the reverse phase column. 10mM of phosphate buffer (pH6.7; the pH may slightly vary since it was prepared by diluting 0.1M phosphate buffer (pH6.7)) and 5% MeOH eluent (solution B) were used. The column temperature was 40°C and the flow rate was 0.8ml/min. The separation pattern is shown in FIG. 5. The separation pattern was obtained using an electrochemical detector ("ESA Coulochem II" from ESA, Inc.) (voltage: 350mV in guard cell; 150mV at channel 1; 300mV at channel 2).

### (Example 3)

### <Purification by anion-exchange chromatography>

Another example of a separation pattern of the human urine sample 2 is shown in FIG. 6. 20µl of human urine sample 2 was injected. As the separation condition, a column MCI GEL CA08F (7µm) (internal diameter 1.5mm, guard column length 5cm, and main column length 15cm) was used. The column temperature was 65°C, and the flow rate was 36µl/min. 0.3mM sulfuric acid, 2% acetonitrile eluent was used as a solution A. The separation pattern was obtained using the UV detector ("UV-8020" manufactured by Tosoh Corp.) (absorption wavelength 254nm).

### (Example 4)

### <Purification by reverse phase chromatography>

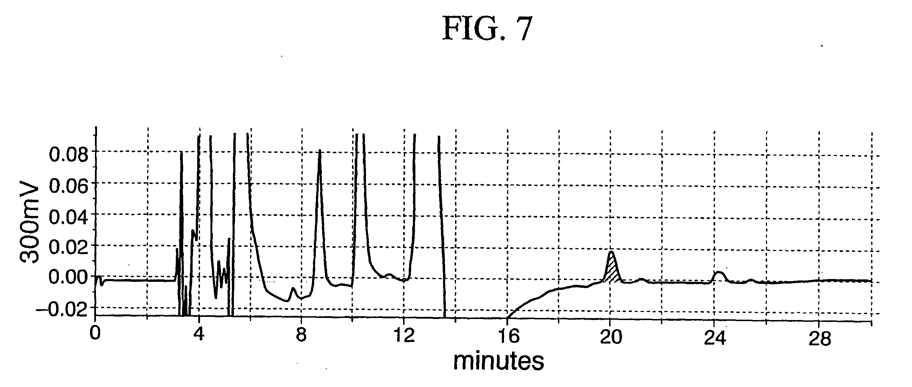

FIG. 7 shows another example where the fraction containing 8-OH-dG obtained by the anion-exchange chromatography was analyzed by reverse phase chromatography. A YMC-Pack ODS-AM (S-5µm) (250x4.6mm) was used for the reverse phase column. 10mM of phosphate buffer (pH7.2; the pH may slightly vary since it was prepared by diluting 0.1M phosphate buffer (pH7.2)) containing 5% MeOH (solution B) was used. The column temperature was 40°C and the flow rate was 0.9ml/min. The separation pattern was obtained using the electrochemical detector ("ESA Coulochem II" from ESA, Inc.) (voltage: 350mV in guard cell; 150mV at channel 1; 300mV at channel 2).

As is clear from FIG. 5 and FIG. 7, according to the method described above, no impurity was found in the vicinity of the purified 8-OH-dG, showing superiority in the accuracy of measuring 8-OH-dG.

### (Example 5)

### <Preparation of urine sample 3>

1ml of rat urine was placed in each of two Eppendorf tubes and frozen at -20°C. The frozen urine was thawed and homogenized. 100µl of each homogenate was diluted with the same volume of slightly acidic solution (composition; 96ml of 0.6mM sulfuric acid and 4ml of acetonitrile). Consequently, 12µg of 8-OH-rGuo was added. The pH was adjusted below 7 by adding 6.7µl of 2M sodium acetate (pH4.5). The mixture was well stirred and centrifuged at 15,000 rpm for 5 minutes. The supernatant was made the urine sample 3.

### <Purification by anion-exchange chromatography>

The rat urine sample 3 was purified using an anion-exchange column to obtain a fraction containing 8-OH-dG. 0.3mM sulfuric acid, 2% acetonitrile eluent was used as a solution A. The column temperature was 65°C and the flow rate was 17µl/min. The anion-exchange column was made using a filler of an Aminex HPX-72S column (manufactured by Bio-Rad) (300x7.8mm, particle diameter 12µm, degree of cross-linkage 8%, and sulfate type) refilled in a column of an internal diameter of 1mm (guard column length 5cm, and main column length 15cm).

### <Purification by reverse phase chromatography>

The fraction containing 8-OH-dG obtained by the anion-exchange chromatography was automatically injected into and analyzed by a reverse phase column. The separation pattern is shown in FIG. 8. A YMC-Pack ODS-AM (S-5µm) (300x4.6mm) was used for the reverse phase column. 10mM of phosphate buffer (pH7.2; the pH may slightly vary since it was prepared by diluting 0.1M phosphate buffer (pH7.2)) containing 5% MeOH (solution B) was used. The column temperature was 40°C and the flow rate was 0.9ml/min. The separation pattern was obtained using the electrochemical detector ("ESA Coulochem II" from ESA, Inc.) (voltage: 350mV in guard cell; 150mV at channel 1; 300mV at channel 2).

### (Example 6)

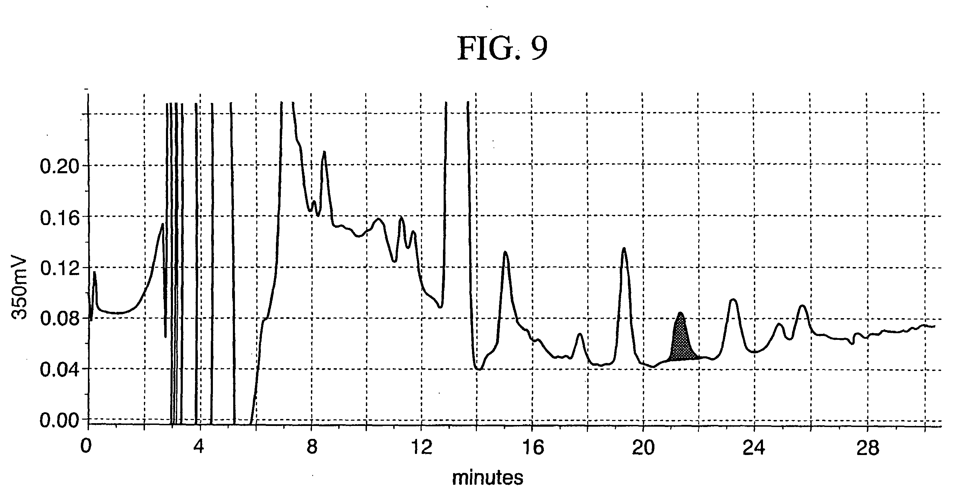

FIG. 9 shows another example where the fraction containing 8-OH-dG obtained from rat urine by the anion-exchange chromatography (MCI, 7µm particle) was analyzed by reverse phase chromatography. A Shiseido Capcell Pak C18 MG (S-5µm)(250x4.6mm) was used for the reverse phase column. 10mM of phosphate buffer (pH6.0; the pH may slightly vary since it was prepared by diluting 0.1M phosphate buffer (pH6.0)) containing 2% MeOH (solution B) was used. The column temperature was 46°C and the flow rate was 0.75ml/min. The separation pattern was obtained using the electrochemical detector ("ESA Coulochem II" from ESA, Inc.) (voltage: 400mV in guard cell; 280mV at channel 1; 350mV at channel 2).

As is clear from FIG. 8 and FIG. 9, it was also possible to measure 8-OH-dG in the rat urine sample 3.

### INDUSTRIAL APPLICABILITY

According to the purification method by anion-exchange chromatography of the present invention, oxidatively damaged guanine nucleosides such as 8-OH-dG, 8-OH-rGuo or the like can be easily purified and recovered with a high recovery rate.

Moreover particularly in the purification of 8-OH-dG, by previously adding the 8-OH-rGuo to the sample as the internal standard marker for 8-OH-dG, the accurate elution time of 8-OH-dG can be obtained so that the fraction containing 8-OH-dG can be reliably collected. Furthermore, since the flow rate of the anion-exchange column (HPLC-1) in the first purification step is very low, the consumption of the eluent (solution A) and the washing solution (solution C) is extremely small, and the amount of the effluent waste after purification is small, so that the method is also preferable from the aspect of environmental protection. Moreover, since the measuring method of the present invention measures the purified oxidatively damaged guanine nucleosides such as purified 8-OH-dG, 8-OH-rGuo or the like, obtained by the above purification method, it has high accuracy and reproducibility. According to the 8-OH-dG analyzer of the present invention, the anion-exchange column (HPLC-1) 11 specifically absorbs 8-OH-dG contained in the sample, increasing the recovery rate and removing almost all impurities contained in the sample at once. Moreover, by continuous operation a large amount of samples can be treated. Since the analyzer is relatively low in price, it is superior in terms of economic efficiency.

## Claims

1. A purification method for oxidatively damaged guanine nucleosides generated as a result of guanine damage in DNA or RNA, comprising a first purification step for purifying oxidatively damaged guanine nucleosides contained in a sample by anion-exchange chromatography.

2. A purification method for oxidatively damaged guanine nucleosides according to claim 1, wherein said oxidatively damaged guanine nucleoside is 8-hydroxydeoxyguanosine (8-OH-dG).

3. A purification method for 8-hydroxydeoxyguanosines (8-OH-dG) contained in a sample, wherein 8-hydroxyguanosines (ribonucleosides) (8-OH-rGuo) are previously added to the sample as an internal standard marker for 8-OH-dG so as to purify it.

4. A purification method for 8-OH-dG (8-OH-dG) contained in a sample, wherein 8-hydroxyguanosine (ribonucleosides) (8-OH-rGuo) is previously added to the sample, comprising a first purification step for purifying said sample by anion-exchange chromatography, and a second purification step for further purifying the fraction containing 8-OH-dG obtained in the first purification step by reverse phase chromatography.

5. A purification method for oxidatively damaged guanine nucleosides according to claim 1 or claim 2, wherein said sample is urine.

6. A purification method for 8-hydroxydeoxyguanosines (8-OH-dG) according to claim 3 or claim 4, wherein said sample is urine.

7. A measuring method for oxidatively damaged guanine nucleosides comprising a measuring step for measuring purified oxidatively damaged guanine nucleosides obtained by the purification method of any one of claim 1, claim 2 and claim 5.

8. A measuring method for 8-OH-dG comprising a measuring step for measuring purified 8-hydroxydeoxyguanosines (8-OH-dG) obtained by the purification method of any one of claim 3, claim 4 and claim 6.

9. A measuring method for 8-OH-dG according to claim 8, wherein said purified 8-hydroxydeoxyguanosines (8-OH-dG) are measured in anion-exchange chromatography in the order of;
(1) peak recognition of ribonucleosides 8-OH-rGuo,
(2) starting of 8-OH-dG fractionation after a fixed time,
(3) finishing of 8-OH-dG fractionation after a fixed time, and
(4) optionally mixing 8-OH-dG fractions, and then injected into a reverse phase column.

10. An apparatus for purifying and measuring 8-hydroxydeoxyguanosines (8-OH-dG), comprising;
an anion-exchange column (HPLC-1) which specifically absorbs 8-OH-dG contained in a sample,
a UV detector which detects an elution position of 8-hydroxyguanosine (ribonucleoside) (8-OH-rGuo),
a reverse phase column (HPLC-2) which further purifies the fraction containing 8-OH-dG obtained from the anion-exchange column (HPLC-1), and
a detector which measures the purified 8-OH-dG obtained from the reverse phase column (HPLC-2).

11. A program for controlling a process for recovering 8-hydroxydeoxyguanosines (8-OH-dG) contained in a sample by column chromatography, which executes on a computer processes for:
receiving a peak signal of a marker (8-OH-rGuo) previously added to the sample from a UV detector;
outputting a signal to open a valve connected to a sampler, during 8-OH-dG elution after a fixed time;
starting fractionation; and
outputting a fractionation termination signal after another fixed time;
and then outputting a signal to inject the obtained 8-OH-dG fraction into a second purifying column;
thereby purifying and recovering a detected substance (8-OH-dG) eluted from the column.
